# EUROPEAN PATENT SPECIFICATION

(11) **EP 2 714 178 B1**
(45) Date of publication and mention of the grant of the patent: **12.08.2015**
(21) Application number: 12722025.9
(22) Date of filing: 08.05.2012
(51) Int. Cl.: A61M 25/098, A61M 25/14, A61M 25/10

(54) **ACTIVE PLEURODESIS CATHETER**
AKTIVER PLEURODESEKATHETER
CATHÉTER DE PLEURODÈSE ACTIVE

(30) Priority: 26.05.2011 US 201113116833
(43) Date of publication of application: 09.04.2014
(73) Proprietor: Carefusion 2200, Inc., San Diego, CA 92130 (US)
(72) Inventor: LANDSMAN, Kelly, Milwaukee, WI 53207 (US); KRUEGER, John, A., Muskego, WI 60085 (US)
(74) Representative: Potter Clarkson LLP
(86) International application number: PCT/US2012/036898
(87) International publication number: WO 2012/161954

(56) References cited:
- WO-A1-2004/098040
- WO-A1-2009/127216
- US-A1- 2002 111 601
- US-A1- 2007 123 825
- US-A1- 2008 172 042
- US-A1- 2009 240 202

## Description

### TECHNICAL FIELD

Embodiments of the present invention relate to the field of removing peritoneal ascites, pleural effusion fluids, and the like. More particularly, embodiments of the present invention relate to a catheter device that can perform a drainage function and an alternate action using the same catheter in an activated configuration.

### BACKGROUND

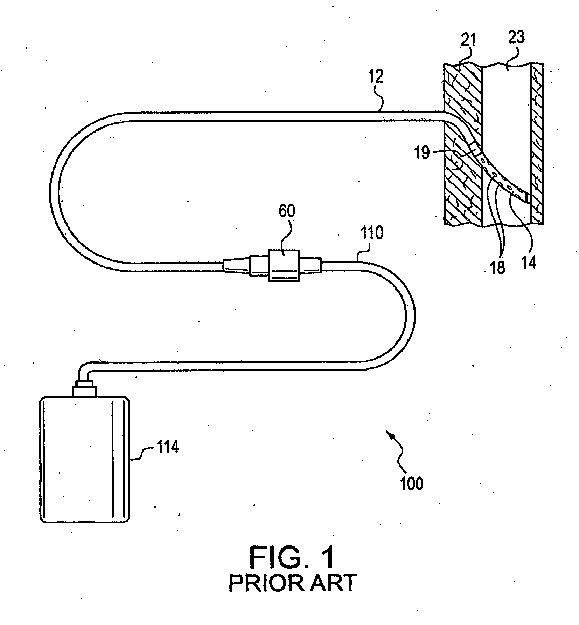

Ascites describes an accumulation of fluid in the peritoneal cavity. Pleural effusion refers to the effusion of fluid into the pleural space. Both excess fluid accumulation conditions may be treated with a drainage apparatus of the type shown in FIG. 1. The apparatus 100 is shown as installed in a patient body and includes a drainage container 114. The drainage container 114 is removably attached by a proximal tube 110 at a valve 60 to a distal catheter 12. The valve 60 may be configured in any number of ways known in the art for attaching catheters together in a fluid-patent manner, (which may include a two-part valve), and the proximal portion attached to the distal catheter 12 may be configured to be self-sealing when disconnected from the proximal tube 110. The proximal end portion of the distal catheter 12 is shown indwelling the patient, disposed through the body wall 21 into an intra-body space 23, which may be - for example - a pleural, peritoneal, or other body lumen. That proximal portion includes a sealing cuff 19 and a flexible fluid-intake length 14 including apertures 18, shown in the intra-body space 23. This structure may be better understood with reference to U.S. Pat. No. 5,484,401 and with reference to commercial products marketed under the name PleurX by CareFusion® of San Diego, Calif.

The pleural space normally contains approximately 5 to 20 ml of fluid. The pH, glucose and electrolytes of the fluid are equilibrated with plasma, but the fluid is relatively protein-free. The fluid is the result of the hydrostatic-oncotic pressure of the capillaries of the parietal pleura. About 80-90% of the fluid is reabsorbed by the pulmonary venous capillaries of the visceral pleura, and the remaining 10-20% is reabsorbed by the pleural lymphatic system. The turnover of fluid in the pleural space is normally quite rapid-roughly 35 to 75% per hour, so that 5 to 10 liters of fluid move through the pleural space each day.

A disruption in the balance between the movement of fluid into the pleural space and the movement of fluid out of the pleural space may produce excessive fluid accumulation in the pleural space. Such disruptions may include, for example, (1) increased capillary permeability resulting from inflammatory processes such as pneumonia, (2) increased hydrostatic pressure as in congestive heart failure, (3) increased negative intrapleural pressure as seen in atelectasis (partial or total lung collapse), (4) decreased oncotic pressure as occurs in the nephrotic syndrome with hypoalbuminemia, and (5) increased oncotic pressure of pleural fluid as occurs in the inflammation of pleural tumor growth or infection. Pleural effusion is particularly common in patients with disseminated breast cancer, lung cancer or lymphatic cancer and patients with congestive heart failure, but also occurs in patients with nearly all other forms of malignancy.

The clinical manifestations of pleural effusion include dyspnea, cough and chest pain which diminish the patient's quality of life. Although pleural effusion typically occurs toward the end of terminal malignancies such as breast cancer, it occurs earlier in other diseases. Therefore relieving the clinical manifestations of pleural effusion is of a real and extended advantage to the patient. For example, non-breast cancer patients with pleural effusion have been known to survive for years.

There are a number of treatments for pleural effusion. If the patient is asymptomatic and the effusion is known to be malignant or paramalignant, treatment may not be required. Such patients may develop progressive pleural effusions that eventually do produce symptoms requiring treatment, but some will reach a stage where the effusions and reabsorption reach an equilibrium that is still asymptomatic and does not necessitate treatment.

Pleurectomy and pleural abrasion is generally effective in obliterating the pleural space and, thus, controlling the malignant pleural effusion. This procedure is done in many patients who undergo thoracotomy for an undiagnosed pleural effusion and are found to have malignancy, since this would prevent the subsequent development of a symptomatic pleural effusion. However, pleurectomy is a major surgical procedure associated with substantial morbidity and some mortality. Therefore, this procedure is usually reserved for patients with an expected survival of at least several months, who are in relative good condition, who have a trapped lung, or who have failed a sclerosing agent procedure.

In general, systemic chemotherapy is disappointing for the control of malignant pleural effusions. However, patients with lymphoma, breast cancer, or small cell carcinoma of the lung may obtain an excellent response to chemotherapy. Another approach to removing fluid from the pleural space has been to surgically implant a chest tube. Such tubes are commonly quite rigid and fairly large in diameter and are implanted by making a surgical incision and spreading apart adjacent ribs to fit the tube into place. Such procedures are painful to the patient, both initially when the chest tube is inserted and during the time it remains within the pleural space.

Thoracentesis is a common approach to removing pleural fluid, in which a needled catheter is introduced into the pleural space through an incision in the chest cavity and fluid is positively drawn out through the catheter using a syringe or a vacuum source. The procedure may also include aspiration utilizing a separate syringe. There are a number of difficulties in thoracentesis, including the risk of puncturing a lung with the catheter tip or with the needle used to introduce the catheter, the risk of collapsing a lung by relieving the negative pressure in the pleural space, the possibility of aggravating the pleural effusion by stimulating fluid production in the introduction of the catheter, and the risk of infection. One of the primary difficulties with ordinary thoracentesis procedures is that fluid reaccumulates in the pleural space relatively quickly after the procedure is performed, and so it is necessary to perform the procedure repeatedly-as often as every few days.

Modern pleural and peritoneal drainage systems have made it possible for patients to use devices like those illustrated in FIG. 1 to conduct drainage on periodic office or hospital visits. For patients who experience recurrent effusions, repeat drainage procedures at a clinical facility can be avoided by the installation of an indwelling tunneled catheter that can be drained at home. In addition, for some patients it may be desirable to administer a substance or provide a therapeutic intervention to the area where the catheter is inserted. For example, in patients with pleural effusion who have a lung that re-expands upon drainage, fusion of the visceral and parietal pleura is a treatment option that eliminates at least a portion of the pleural cavity and thus eliminates the space where the fluid accumulates. This procedure is called pleurodesis and can be accomplished through inciting the patient foreign body response and draining the effusion. Mechanical or chemical means can be used to cause the irritation. In other instances, continuous delivery of medication or cell signaling molecules may be desired in the area where the catheter resides.

Chemical pleurodesis may use irritants and/or antibiotic materials that may also provide mechanical irritation to trigger cell growth and/or resist infection. Examples of materials known and used include bleomycin, tetracycline, and povidone iodine. As another example, a slurry of talc can be introduced into the pleural space. These materials generally are introduced through a thoracic drainage catheter. The instilled chemicals cause irritation between the parietal and the visceral layers of the pleura which closes off the space between them and prevents further fluid from accumulating. Chemical pleurodesis may be a painful procedure, so patients are often premedicated with a sedative and analgesics. A local anesthetic may be instilled into the pleural space, or an epidural catheter may be placed for anesthesia. Generally, to be effective, introduction of structures and materials for pleurodesis desirable will create irritation and then keep the space dry. In order to establish pleurodesis, it is preferable that the parietal and visceral layers of the pleura remain in juxtaposition. As such, it is preferable that when mechanical and/or chemical irritation is complete a drainage tube will remain in place to remove the fluid over the time it takes for the adhesion accomplishing pleurodesis to occur.

Accordingly it may be desirable to provide a catheter that can be placed in a body cavity for drainage of pleural or peritoneal fluids or the like, and then be converted *in situ* to perform a secondary function. Specifically, embodiments described herein may provide dual functionality of both stimulating pleurodesis and providing for drainage until pleurodesis is established. Development of such a device reduces the number of interventions required on the patient, thus minimizing the risk of infection, while also allowing normally inpatient procedures to be performed in an outpatient manner.

### BRIEF SUMMARY

According to the present invention, there is provided a dual state catheter device according to claim 1.

Embodiments of this technology may be used to break up loculations (multiple small spaces/ cavities), to prevent catheter migration, and/or disrupt a fibrin sheath. Some embodiments may be useful to prevent or minimize biofilm formation on catheter and/or other tube surfaces associated with different embodiments of the presently disclosed device.

### BRIEF DESCRIPTION OF THE DRAWINGS

FIG. 1 illustrates a drainage apparatus as known in the prior art;
FIG. 2 shows a comparative distal indwelling portion of a dual-state catheter;
FIGS. 3A and 3B show unactuated and actuated states, respectively, of an embodiment of a dual-state catheter;
FIGS. 4A-4E show the components and actuation of another comparative dual-state catheter;
FIGS. 5A and 5B show unactuated and actuated states, respectively, of another comparative dual-state catheter;
FIGS. 6A and 6B show unactuated and actuated states, respectively., of another comparative dual-state catheter;
FIGS. 7A-7C show the components and actuation of another comparative dual-state catheter; and
FIGS. 8A-8D show the components and actuation of another embodiment of a dual-state catheter.

### DETAILED DESCRIPTION

Embodiments generally are described with reference to the drawings in which like elements are generally referred to by like numerals. The relationship and functioning of the various elements of the embodiments may better be understood by reference to the following detailed description. However, embodiments are not limited to those illustrated in the drawings. It should be understood that the drawings are not necessarily to scale, and in certain instances details may have been omitted that are not necessary for an understanding of embodiments of the present invention, such as-for example-conventional fabrication and assembly.

The present invention now will be described more fully hereinafter. This invention may, however, be embodied in many different forms and should not be construed as limited to the embodiments set forth herein; rather, these embodiments are provided so that this disclosure will be thorough and complete, and will fully convey the scope of the invention to those skilled in the art. As used in this specification and the claims, the singular forms "a," "an," and "the" include plural referents unless the context clearly indicates otherwise. Throughout the specification, the terms "distal" and "distally" shall denote a position, direction, or orientation that is generally away from the physician and/or toward the patient. Accordingly, the terms "proximal" and "proximally" shall denote a position, direction, or orientation that is generally towards the physician and/or away from the patient.

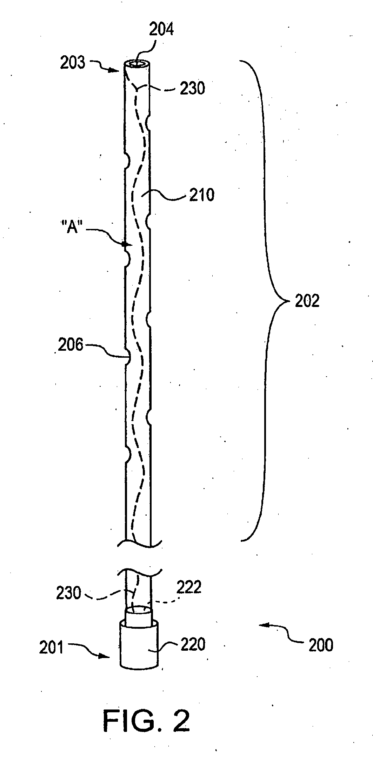

FIG. 2 shows a distal indwelling portion 202 of a dual-state catheter 200. The catheter 200 has a proximal end region 201 and a distal end region 203. The catheter includes an inner lumen 204 extending between the proximal and distal ends 201, 203. The distal indwelling portion 202 of the catheter 200 is fenestrated with a series of holes 206 allowing fluid communication between the exterior of the distal indwelling portion 202 and the lumen 204 (e.g., for drainage). The proximal end of the catheter is attached to a valve 220. The valve 220 includes an inner valve surface 222. The catheter includes conducting means 230 (which may be embodied, e.g., as embedded wires, semiconductor filaments, fiber optic lines, coextruded conducting stripe, silver impregnated material, liquid channel, overmolded material, or any combination thereof) that extend from the proximal end region 201 into the indwelling portion 202 of the catheter 200. The conducting means 230 preferably are configured to carry/transmit current and/or other forms of energy (e.g., RF energy, microwave, UV, infrared) to the distal indwelling portion 202 of the catheter 200 and to apply that energy to the outer surface 210 of the distal indwelling portion 202. The catheter 200 may be constructed including flexible material such as silicone rubber, molded or extruded polymer, or alloy material.

The outer surface 210 of the distal indwelling portion 202 of the catheter 200 is coated with a substance "A." In a first (non-activated) state, the substance "A" preferably is non-functional and non-toxic. One such example may include an electrolyte solution suspended or otherwise carried in a polymeric or nonpolymeric material on the catheter surface. Some materials suitable for use may include impregnated meltblown. materials, hydrogel, dip coating materials, and/or resin impregnation materials. The catheter 200 may be configured to remain in a closed state (e.g., where the lumen 204 does not have free/open communication through the proximal catheter end 203) when not being used, by means of valve 220.

The catheter 200 may be converted to a second state by attaching it to source of energy (e.g., microwave, electrical current, UV, RF, infrared, heat, etc. including any combinations of different kinds of energy). The energy may be transmitted to the conducting means 230 and thereby applied to the distal indwelling portion 202 of the catheter 200. The valve 220 may include an interface configured to provide connection between an energy source and the conducting means 230. In one arrangement, the energy applied may be electrical current. Application of the current causes a coating "A" to be converted to "A++" (by means of electrical separation, e.g., anode/cathode effect). In such an arrangement, the coating "A++" is configured to perform a desired clinical therapeutic function. Where the coating "A" of the distal indwelling portion 202 is configured as an electrolyte salt solution, when DC (separating) current is applied, electrolyzed water may be formed and released near the outer surface 210 of the distal indwelling portion 202. Electrolyzed water, which is slightly acidic (e.g., about 6.5 pH) is known to provide strong antimicrobial properties. This invention can be applied to a variety of substances that are known to be inert/non-toxic in an initial state and that can be converted to a functional state through application of a selected type of energy. For example, a variety of proteins, other complex organic molecules, ionic solutions, and inorganic materials are known to exist in relatively inert/ inactive states that may be rendered active by application of a selected form of energy.

One example of a material that may be used includes dried talc as coating "A," complexed to a catheter surface by a coating material, and able to be released/activated by a stimulus such as ultrasonic vibration. Generally, an accelerodesis coating complexed to salts or a more permanent bond to the catheter surface may released and/or otherwise activated by the application of energy. Depending upon the type of bond or other connection, the energy may be - for example - mechanical, electrical, or some other kind of energy, or any combination thereof. Those having skill in the art of materials science with application to internal medicine will appreciate a variety of releasable and/or activatable compounds and methods of activatable or otherwise selectable delivery known in the art. For example, may different stents, catheters, and implantable medical devices incorporate bioreactive materials that are released over time, that are activated upon specific stimulus and/or environmental factors, or that are otherwise able to be delivered in a controlled manner.

Those of skill in the art will appreciate that the conducting means 230 may be selected and constructed in view of the type of energy to be used, which will - in turn - be selected in conjunction with the selection of activatable coating material "A." For example, a catheter may be constructed with coating "A" being a gelatinous polymer including a UV-sensitive protein that is inactive/inert until exposed to UV, but which changes configuration to function as a pleurodesis agent upon UV exposure. As such, fiber optics or other materials configured to transmit UV through the catheter body to the coating "A." Upon activation by the selected energy type(s), the coating "A" is transformed from its inert/ inactive state to an active state that will promote pleurodesis.

As described above, a therapeutic ingredient of the coating "A" may be activated by the selected energy type(s). In another variation, the coating "A" may include an active ingredient that is enclosed, encased, or otherwise protected by an inert carrier material such as - for example - a polymer. The inert carrier material can be altered to release the active agent of the coating "A." For example, the inert carrier material may be softened/melted, have pores or micro-passages that are opened/relaxed, or otherwise undergo a chemical and/or mechanical change that will release or otherwise expose the active agent of the coating "A" to an area around the distal catheter portion 202 to promote pleurodesis.

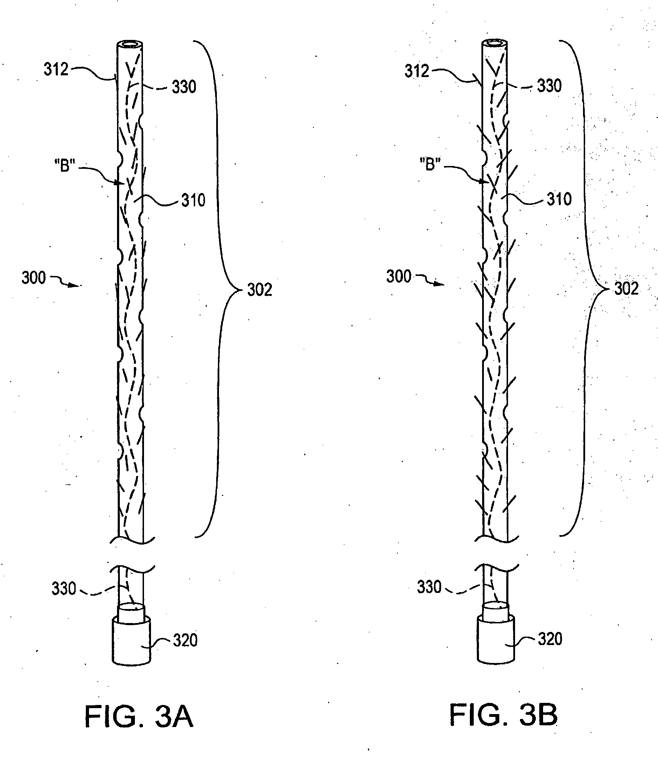

FIGS. 3A and 3B illustrate an alternative embodiment of a dual-state catheter according to the present invention in which a conformational change rather than a chemical change is conveyed by application of energy. The outer surface 310 of the distal indwelling portion 302 of the catheter 300 includes attached bristles 312, which may be coated with, for example, one or more pharmaceutical sclerosing agents, antimicrobial agents, or other materials configured for inducing pleurodesis (e.g., silver nitrate, talc slurry, bleomycin, tetracycline, povidone iodine, or and combination thereof). The outer surface of the entire device, or of just the coated bristles may be over-coated with an energy-releasable or otherwise activatable/releasable coating substance "B," such as, for example, a hydrogel, a soluble gel solution, a starch, an absorbable suture material, a water soluble adhesive, or any combination thereof. In a first, inactivated state, the bristles 312 preferably lay substantially flat to the outer surface 310, providing a low outer profile for the catheter 300, as shown in FIG. 3A. When a clinician desires to initiate therapy that requires mechanical abrasion (such as in pleurodesis) and/or shape change of the pleural space (such as adhesion prevention by spreading apart of adjacent tissue), a selected energy form is communicated from an energy source to the conducting means 330 (e.g., via an interface (not shown) on the valve or other catheter portion). The conducting means 330, in turn, transmits (or at least forms a conduit for communication of) the energy to the outer surface 302 of the distal indwelling portion 302 to activate the coating "B."

In one embodiment, the coating substance "B" may be embodied as an inert polymer, such as a hydrogel, and the energy applied may be microwave energy. The inert polymer may be configured as having a soft and/or tacky composition when in a partially cured form, which soft and/or tacky characteristic decreases or is eliminated by microwave curing. In the partially cured form, the soft and/or tacky trait of the coating substance "B" preferably will adhere or otherwise hold the bristles 312 close to the catheter body in the low profile shown in FIG. 3A. When microwave energy is applied to the catheter 300, the inert polymer of coating "B" will be cured. In some embodiments, the coating may be temperature-sensitive. For example, a hydrogel (stable in at least semisolid form to above body temperature) may be used that will hold down the bristles during introduction of the device, but then application of heat via the conductive structure will liquefy the hydrogel, releasing the bristles. As such, as a general principle of operation, the effect of curing or other effect upon coating "B" preferably will release at least some of the bristles 312 to the outer surface 310 of the distal indwelling portion 302, so that they are extended into a larger circumferential profile from the catheter body as shown in FIG. 3B. The bristles 312 will then be exposed to the tissue.

In an application where mechanical irritation is desired (e.g., for promoting pleurodesis), the catheter may be moved about and/or placed into an orientation that it will move as the patient moves and breathes. This same method of activation may be performed using repulsive charges (e.g., where the coating "B" has a first charged state that promotes the bristles being held in low profile, but which charged state is reversible to allow or cause the bristles to extend/ re-orient to a larger profile state), cleaving of bonds (e.g., where coating "B" provides chemical and/or mechanical bonding between the lengths of bristles and the catheter body, which bonding is released by transmission of selected energy), denaturing of a protein linker used to bond bristle lengths into the low-profile configuration of FIG. 3A, completion of curing via UV (rather than, or in addition to microwave energy as described above), etc. Other coatings may be provided that will swell, fill up a space or re-expand to a predetermined shape (e.g., provide a scaffold for stem cell or tissue growth). In view of the present disclosure, those of skill in the art will be enabled to provide other physical and mechanical variations using materials known in the art, which may be practiced within the scope of the present invention.

In some instances, a coating being used to provide a therapeutic treatment may not be convertible to an active state *in situ*. However, the advantages described above for converting a catheter from an initial state to a second state may still apply. In such instances, it may be desirable for the catheter to include a chemical coating (a pharmaceutical, a sclerosing agent, an antimicrobial, etc., as described above with reference to FIG. 2). At an initial insertion, a clinician may not have sufficient information to administer / expose a chemical agent. However, after the initial intervention (e.g., introduction of a drainage catheter of the present invention that includes an activatable functionality), the information desired (e.g., confirmation of lung expansion, detection of cancerous cells, detection of infection, etc.) may be gathered and/or confirmed, and - if appropriate - the catheter may be activated by introduction of the selected energy type(s) to release or expose therapeutic agents and/or structures.

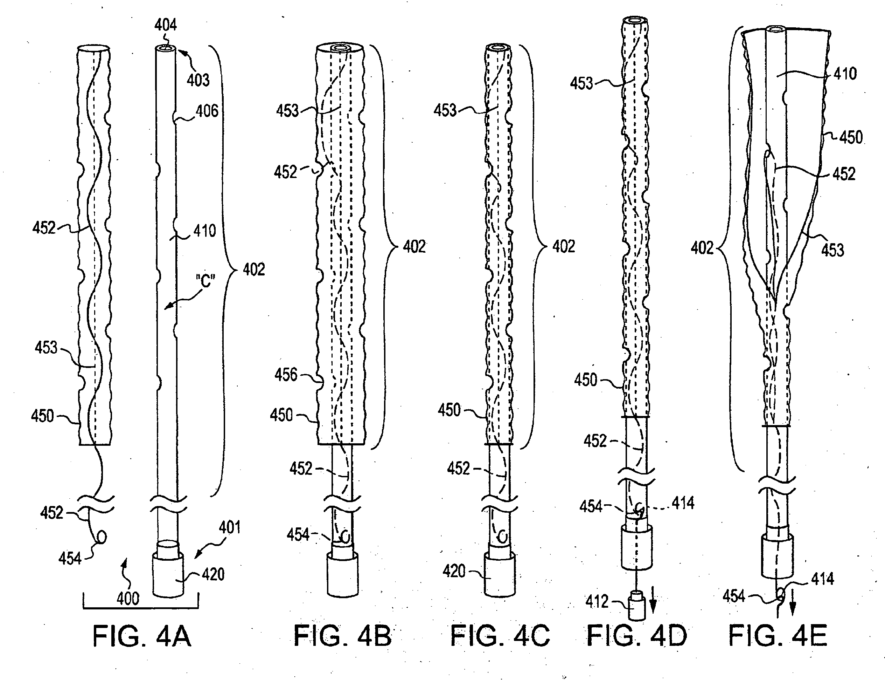

FIGS. 4A-4E show assembly and actuation of another dual-state of a catheter 400 which is configured to transform from a first state into a second state by mechanical actuation. In FIGS. 4A-4E, the catheter 400 includes a proximal end 401, a distal end 403, and a distal indwelling portion 402. The catheter 400 includes an inner lumen 404. At least one fenestration 406 on the outer surface 410 of the distal indwelling portion 402 of the catheter 400 is configured to allow communication between the outer surface 410 of the distal indwelling portion 402 and the inner lumen 404 (e.g., for drainage). The at least one fenestration may include a plurality of fenestrations, and may be configured as a round aperture, slit, or any other shape of opening that those of skill in the art will appreciate as providing for the functionality here described. The proximal end 401 of the catheter 400 is illustrated as being attached to a valve 420.

The outer surface 410 of the distal indwelling portion 402 may be coated with a therapeutic chemical substance "C." Examples of such therapeutic substances may include a sclerosing agent (e.g., talc, silver nitrate, PVP, bleomycin), a chemotherapy agent, an antibiotic, or any combination thereof with each other and/or another suitable material As shown in FIG. 4A, a sheath 450 is configured to encapsulate the distal indwelling portion 402 and prevent the outer surface 410 from making contact with the body cavity surface. The sheath 450 preferably includes a material that the coating "C" cannot penetrate such as, for example, a cellulose compound, low-permeability hydrogel, or a materials such as is used for absorbable suture material.

The sheath 450 preferably has a similar shape to the distal indwelling portion 402 of the catheter 400, and - when assembled thereto - preferably will conform closely to the outer surface 410 of the catheter body. Fenestrations 456 on the sheath 450 may be aligned with fenestrations 406 on the distal indwelling portion 402 as illustrated in FIGS. 4B-4D, such that they may facilitate drainage while the sheath 450 remains closely disposed about the catheter body. The sheath 450 may be constructed with fenestrations 456 (as shown in FIG. 4A), then assembled to the rest of the catheter as shown in FIGS. 4B-4C, or the sheath and catheter fenestrations can be created together after assembly of the sheath and the catheter, which may simplify and facilitate maintaining alignment of sheath fenestrations 456 with catheter fenestrations 406. When the catheter 400 is assembled, the sheath 450 preferably will be secured in a tightly conforming manner to the distal-indwelling portion 402 as shown in FIG. 4C. This may be accomplished in a variety of ways, such as, for example, co-extrusion, heat shrink, adhesive, friction fit, or other methods known in the art or yet to be developed.

The sheath 450 preferably includes means by which it can be split open or otherwise manipulated so as to expose the distal indwelling portion 402. This can be accomplished in a variety of ways. Some examples of how those of skill in the art may provide this function include use of a frangible seal, an imbedded tear-out thread, a tongue and groove connection, one or more pressure sensitive adhesives, a peel pouch, a hook and loop connection, a zipper, one or more buttons, or any other suitable connecting means. One example is illustrated here with reference to FIGS. 4A-4E.

The sheath 450 is shown separate from the catheter body in FIG. 4A. The sheath 450 includes a splittable seam region 453 (illustrated by a generally vertical dashed line, which is not to be confused with the dashed lines used to illustrate underlying structures after assembly, as shown in FIGS. 4B-4E, and which seam 453 may be oriented helically, at an angle, or other orientation). At the time of application of the sheath 450 to the distal indwelling portion 402, the opening means 452 (which may be embodied as a filament, thread, wire, or other elongate structure) may be threaded from the outside of the distal indwelling portion 402 to the inner lumen 404 of the catheter 400. This can be accomplished by moving through a fenestration 406 or through a different opening specifically created for this purpose. The opening means 452, shown here as a peel away wire that is embedded in the sheath 450 along the seam 453 , includes a proximal access loop 454 that may reside in and/or extend through the catheter lumen 404. The loop 454 may be oriented to remain ensconced within the lumen 404 such that, once the distal indwelling catheter portion 402 is placed into the patient, the access loop 454 can only be obtained by going through the valve 420. This will help maintain patency of the indwelling catheter portion 402, and may help ensure that access and removal of the sheath 450 can be done without increasing infection risk or opening the patient body cavity occupied by the catheter.

FIG. 4D illustrates how the access loop 454 can be reached by inserting an access instrument 412 through the valve 420. A hook or other coupling means 414 in the instrument 412 may be used to snare or attach to the loop 454. The catheter 400 can be converted to its second, activated state as shown in FIG. 4E by actuating the access instrument 412 so that the opening means 452 is pulled, splitting the sheath 450 and exposing the therapeutic material "C" on the catheter external surface 410. If desired, the sheath 450 be configured to be completely removed through the catheter 400 by continued pulling, or it may be configures such that it can dissolve in the patient's body, and the opening means 452 be removed through the catheter 400. All or parts of the sheath 450 may be removed depending on the desired use. Removal can be done at a single time point and/or in a single action, it can be done repeatedly over time if the seam 453 allows re-connection, or it may be done slowly - continuously or in steps or stages - over an extended time. The seam 453 may be configured as being continuous through the sheath or it may be configured in sections. The coating "C" on the outer surface 410 of the distal indwelling portion 402 can now come in contact with the body cavity to promote a desired therapeutic function.

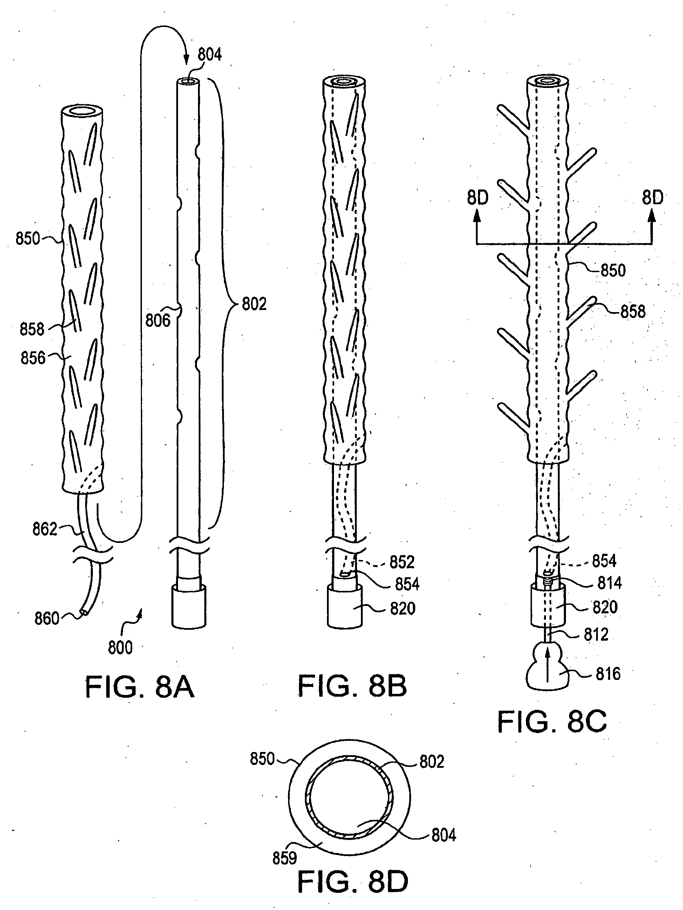

It may be desirable to provide a distal indwelling portion of the catheter that is coated with a substance that is intended to be delivered to the body over an extended period of time in a diluted, consistent, and/or titrated manner. One example of such a system maybe a catheter intended for pleurodesis of the pleural space by means of the sclerosing agent silver nitrate. In these instances, it is preferable that the silver nitrate coating in its base/concentrated form not contact the surrounding tissue directly due to its high concentration and potential tissue reactions thereto. The coating most preferably will be eluted or otherwise be released over time from the catheter. As such, it would be useful to provide a catheter with a sheath that is permeable to body fluids, but that prevents direct contact of the catheter to body surfaces. This can be accomplished in a variety of ways including a coating of differential thickness across the surface of the catheter that may dissolve over time. Additionally, or in the alternative, differing sections of the catheter can be configured to take more fluid exposure to break a bond between a catheter surface and its coating (e.g., each portion of the catheter may be exposed to differing amounts of curing or concentrations of base coating). A catheter could also have a series of "rings" that are shaped like a jellyfish cup/body and coated with silver nitrate on a base/ underlying surface. In the first state, the cup-like bodies are sealed to the catheter (e.g., with a base coating disposed over the entire external surface). A base surface coating dissolves the cup-like or umbrella shape opens exposing the sclerosing agent to the surroundings but not placing it into direct contact with the adjacent tissue. This may be understood with reference to FIGS. 8A-8D, except that one or more of the "bristles/ arms" 858 is embodied as the cup-like or umbrella shape referred to here.

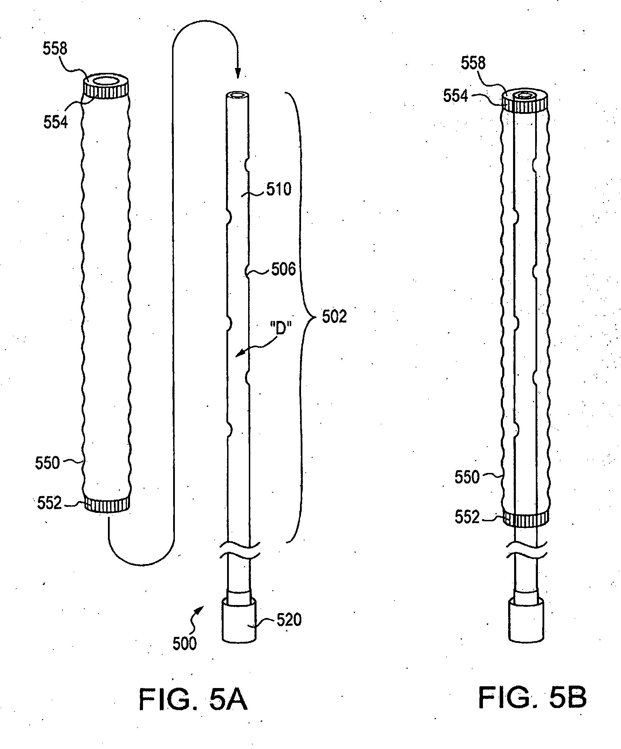

FIGS. 5A-5B illustrate assembly of a catheter device 500 wherein a distal indwelling portion 502 of the catheter 500 is covered by a sheath 550 that is permeable to body fluids, but that prevents direct contact of the catheter outer surface 510 with body surfaces. The sheath may be constructed of, for example silicone, TPU, stainless steel, nylon, implantable foam, polypropylene, urethane, PVC, or other biocompatible material that may be constructed, for example, as a porous or fenestrated tube, a stent-like cage, or other configuration.

FIG. 5A shows the sheath 550 not yet assembled to the catheter 500. The sheath 550 has a proximal end 552 and a distal end 554 that are configured to secure the sheath 550 to the distal indwelling portion 502. Before being covered by the sheath 550, the outer surface 510 of the distal indwelling portion 502 may be coated with a substance "D" (e.g., silver nitrate) for therapy. This design may be combined with various sheath constructions for accelerodesis. For example, in combination with the control hook described above, its shape may be changed to cause or at least promote mechanical abrasion or accelerate dissolving of a coating, or to expose a coating for a brief period of time and then recover/protect it. In such an embodiment the sheath may be configured not to be fluid-permeable.

The sheath 550 will be configured to prevent the outer surface 510 from contacting body surfaces directly. After mounting the proximal end 552 of the sheath 550 over the distal end of the catheter 500, the distal end 554 of the sheath 550 may be secured to the distal end of the catheter 500 by friction fit, adhesive, or other means. Sheath rim 558 covers the edge of the distal end of the sheath 550. If present, fenestrations 506 on the surface of the distal indwelling portion 502 preferably will remain unobstructed. Certain arrangements may be configured with an appearance like wire stents or "hair curler covers" (e.g., as a tube of woven wire or polymer, web-like or mesh-type construction of other biocompatible material). Examples may include a wire mesh tube, a tube of porous polymeric film configured to allow diffusion of a pleurodesis-inducing material, or a woven tube of polymer including apertures. Such a flexible cage-like design may be deformed to be placed into the body, but be configured with resilient enough structure to hold the coated catheter surface away from direct contact with adjacent tissue. The distal indwelling portion 502 covered by the sheath 550, as shown in FIG. 5B, may be placed into the body without the active agent of substance "D" on the outer surface 510 having direct contact with the patient's body.

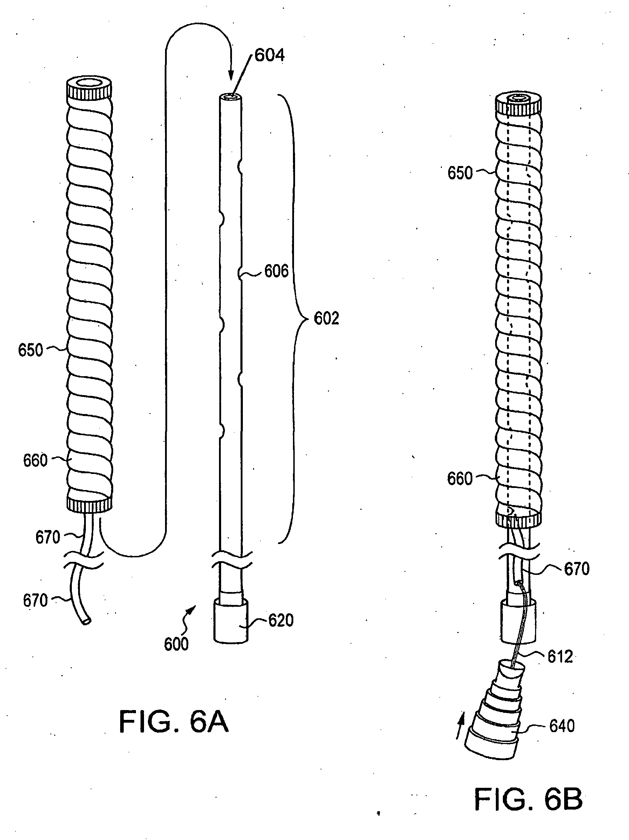

Alternatively, an active agent or activating agent (e.g., an acid or base) can be added to the catheter *in situ*. In order to accomplish this, the catheter surface may be provided with a fluid receiving/retaining portion or this portion may be added externally to the catheter. An arrangement with this feature is described in FIG. 6.

FIGS. 6A-6B illustrate a dual-state catheter device. A sheath 650 including an absorbent portion 660 (e.g., capillary matrix, sponge, hydrogel, dialysis tubing, etc.) is shown in FIG. 6A, and is configured to absorb and slowly release and/or titrate out a therapeutic agent. The absorbent portion 660 preferably is configured to fit on/over a distal indwelling portion 602 of the catheter 600. The sheath 650 includes a filling line 670 that may extend through some or most of the length of absorbent portion 660 in a manner that preferably is configured to evenly supply therapeutic material to the absorbent portion 660 and is also configured to extend down through a central lumen 604 of the catheter body. Once the sheath 650 is assembled to the distal indwelling portion 602, as shown in FIG. 6B, the filling line 670 may be directed into the lumen 604 through a fenestration 606 of the distal indwelling portion 602 or other opening specifically configured for it.

When a clinician determines that a therapeutic agent 640 is desired, the catheter 600 can be converted to its second state by accessing the filling line 670 with an access instrument 612 that may be directed through the valve 620 of the catheter 600. The access instrument 612 may be coupled to a therapeutic agent delivery container 640, and the therapeutic agent may be delivered (e.g., by injection) into the sheath 650 and distributed throughout the sheath absorptive surface 660 via the filling line 670. Thus, the second/active state of the sheath 650 is when it includes and is capable of delivering a therapeutic agent. In this second state, the catheter 600 preferably will provide delivery of the agent to the body as determined by the properties of the sheath absorptive surface 660 material. Therapeutic agents may include silver nitrate liquid, one or more antibiotics, protein rich solutions, an acid or base configured to activate a component in the sheath material, and/or a talc slurry - any or all of which may be delivered using a modified syringe and needle system. In certain arrangements the sheath could be configured as a container with a "slow leak" that a scelerosing agent is instilled into such that the container could release sclerosing agent over time and/or the "leak" could be activated when the catheter is drained by means of a Venturi type of effect.

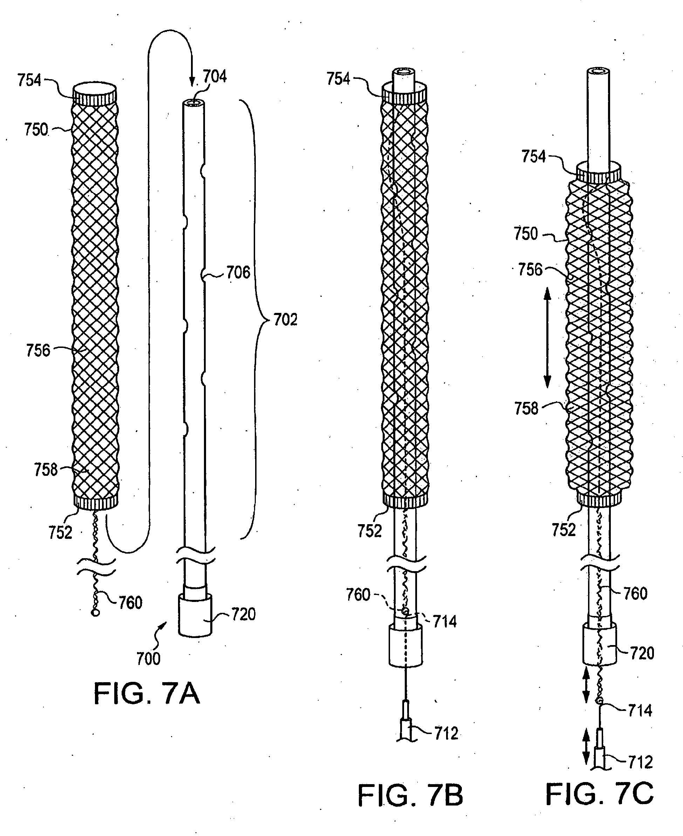

Certain therapies exist wherein causing irritation to a tissue to incite an inflammatory response is beneficial, as in a pleurodesis procedure. FIG. 7 illustrates a dual-state catheter device which is configured to provide mechanical abrasion to the pleural surface in a manner different than the bristle embodiment of FIGS. 3A-3B. In a preferred arrangement, a sheath 750 includes a woven and/or braided filamentous structure 758 that includes a proximal end 752 and a distal end 754 as shown in FIG. 7A. The woven and/or braided structure 758 preferably is constructed to be radially expandable/contractable. The distal end 754 may be configured to maintain the rigid shape of the sheath 750. The sheath 750 is shown in FIG. 7B as installed upon and attached to a fenestrated distal indwelling portion 702 of the catheter 700 at the sheath's proximal end 752. The threads, wires, or other elongate elements that make up the filaments 756 of the woven/braided filamentous structure 758 of the sheath 750 are attached to, or extend continuously with an access string 760. The access string 760 is threaded via a fenestration 706 of the distal indwelling portion 702 (or another specifically constructed opening) through the inner lumen 704 that extends through catheter 700.

When mechanical abrasion is desired, the catheter 700 can be converted/activated to its second state by accessing the string 760 through the catheter valve 720 by means of an access instrument 712. The access instrument 712 may be pushed through the valve 720 and a hook 714 (or other structure) of the access instrument 712 may be advanced and connected to the access string 760. After it is engaged with the access string, the hook 714 of the access instrument 712 may be reciprocally actuated relative to the catheter body. This preferably will actuate the woven/braided structure 758 of the sheath 750 to contract and expand in diameter, with some foreshortening, as shown in FIG. 7C-thus causing mechanical abrasion to surrounding tissue. Thus, in the first state, the filamentous sheath 750 generally or completely closely conforms to the outer surface of the distal catheter portion 702, and - in the second state - the filamentous sheath 750 is expanded to a greater diameter than the catheter. Woven and braided structures for effecting this are well known in the stent art, and those of skill in the art will readily use and/or adapt such structures for use in accord with the present disclosure.

Alternatively, or in addition, the abrasion may be provided by expanding the sheath and moving the entire catheter assembly 700 (e.g., longitudinally, rotatingly, side-to-side). The sheath 750 design is not limited to a woven structure. Any design which can be expanded/ contracted by reciprocal movement of a sheath actuator portion may be practiced within the scope of the present disclosure.

Another embodiment of a dual-state catheter configured for providing mechanical abrasion is illustrated in FIG. 8. A sheath 850 is provided as a tube that can be placed over a distal indwelling portion 802 of a catheter 800, which includes a longitudinal lumen 804 therethrough. The tube sheath 850 includes a fluid-patent internal lumen 859 configured as a generally cylindrical tube lumen that configured to be filled with and contain an inflation/expansion fluid (that may be liquid and/or gas). The generally cylindrical tube lumen 859 may best be understood with reference to FIG. 8D, which is a transverse section view along line 8D-8D of FIG. 8C. The tube 850 preferably is shaped to have multiple extending "arms" or bristles 858 that, when deflated/ inactive in a first catheter state, will lie substantially flat relative to the surface 856 of the tube as shown in FIG. 8A.

The internal lumen 859 of the sheath 850 is configured to be filled through a port 860. The port 860 is connected in fluid communication with the sheath lumen 859 by a fluid line 862. The fluid line 862 may be disposed through a fenestration 806 of the distal indwelling portion 802 or through an opening specifically configured for it, so that it is internal to the catheter 800 as shown in FIG. 8B. The port 860 may be bonded to the catheter valve 820, so that it can be accessed. When mechanical abrasion is desired, the catheter 800 can be converted to its second state. An access instrument 812 may be inserted into the valve 820 and its tip 814 on the access instrument 812 mated with the port 860 as shown in FIG. 8C. A fluid source 816 may attached to the access instrument 812 and fluid directed into the sheath 850. Addition of fluid to the sheath 850 will causes the "arms" 858 to extend as the sheath lumen 859 becomes turgid. One or more of the arms/bristles 858 may include internal lumen(s) in fluid communication with the generally cylindrical tube lumen 859, such that they may become partially or wholly turgid to extend out from the sheath body in a manner forming an expanded diameter greater than that of the catheter body. Mechanical abrasion may be effected by this expansion and/or by subsequent movement of the now-expanded/activated catheter 800.

Those of skill in the art will appreciate that embodiments not expressly illustrated herein may be practiced within the scope of the present invention, including that features described herein for different embodiments may be combined with each other and/or with currently-known or future-developed technologies while remaining within the scope of the claims presented here. Although specific terms are employed herein, they are used in a generic and descriptive sense only and not for purposes of limitation. It is therefore intended that the foregoing detailed description be regarded as illustrative rather than limiting. Furthermore, the advantages described above are not necessarily the only advantages of the invention, and it is not necessarily expected that all of the described advantages will be achieved with every embodiment of the invention.

## Claims

1. A dual state catheter device (300, 800) comprising:
an elongate outer tubular body (850) comprising
an inner tube disposed longitudinally through the outer tubular body;
a distal portion (302, 802) comprising
a distal end configured for insertion into a body cavity,
an outer surface (310, 856), and
at least one fenestration (806) on the outer surface;
a proximal end configured for connection to a valve (320, 820);
a conductive structure (330) configured to carry energy through at least a portion of the outer tubular body; and
bristles (312, 858);
wherein the catheter device is configured to effect a change from a first state to a second state by introduction of a selected energy into the conductive structure, and
wherein the first state of which comprises the bristles lying substantially flat to the outer surface and the second state of which is **characterized by** a mechanical change comprising the bristles extending out from a circumferential profile of the outer surface.

2. The dual state catheter device of claim 1, wherein the conductive structure (330) is selected from embedded wires, semiconductor filaments, fiber optic lines, coextruded conducting stripe, silver impregnated material, liquid channel, overmolded conductive material, and any combination thereof.

3. The dual state catheter device of claim 1, wherein the bristles (312, 858) comprise material configured for inducing pleurodesis.

4. The dual state catheter device of claim 3, wherein the material configured for inducing pleurodesis is selected from a group consisting of silver nitrate, talc slurry, bleomycin, tetracycline, povidone iodine, and any combination thereof.

5. The dual state catheter device of claim 1, wherein the bristles (312, 858) in the first state are held to the outer surface by a releasable coating substance selected from hydrogel, soluble gel solution, starch, absorbable suture material, water soluble adhesive, denaturable protein, and any combination thereof.

## Patentansprüche

1. Doppelzustandskatheter-Vorrichtung (300, 800), Folgendes umfassend:
einen länglichen äußeren röhrenförmigen Körper (850), der Folgendes umfasst:
eine innere Röhre, die in Längsrichtung durch den äußeren röhrenförmigen Körper hindurch angeordnet ist;
einen distalen Abschnitt (302, 802), der Folgendes umfasst:
ein distales Ende, konfiguriert zum Einführen in eine Körperhöhle;
eine äußere Oberfläche (310, 856); und
wenigstens eine Fenestration (806) an der äußeren Oberfläche ein proximales Ende, konfiguriert zum Verbinden mit einem Ventil (320, 820);
eine leitfähige Anordnung (330), konfiguriert, um Energie durch wenigstens einen Abschnitt des äußeren röhrenförmigen Körpers zu transportieren; und
Borsten (312, 858);
wobei die Kathetervorrichtung konfiguriert ist, um einen Wechsel von einem ersten Zustand in einen zweiten Zustand durch Einleiten einer ausgewählten Energie in die leitfähige Anordnung durchzuführen, und
wobei der erste Zustand beinhaltet, dass die Borsten im Wesentlichen flach an der äußeren Oberfläche anliegen und der zweite Zustand durch einen mechanischen Wechsel gekennzeichnet ist, der beinhaltet, dass die Borsten von einem umlaufenden Profil der äußeren Oberfläche abstehen.

2. Doppelzustandskatheter-Vorrichtung nach Anspruch 1, wobei die leitfähige Anordnung (330) ausgewählt ist aus integrierten Drähten, Halbleiterfilamenten, faseroptischen Leitungen, coextrudiertem leitfähigem Streifen, mit Silber imprägniertem Material, Flüssigkeitskanal, umspritztem leitfähigem Material und einer beliebigen Kombination daraus.

3. Doppelzustandskatheter-Vorrichtung nach Anspruch 1, wobei die Borsten (312, 858) Material enthalten, das konfiguriert ist, eine Pleurodese einzuleiten.

4. Doppelzustandskatheter-Vorrichtung nach Anspruch 3, wobei das Material, das zum Einleiten einer Pleurodese konfiguriert ist, ausgewählt ist aus einer Gruppe bestehend aus Silbernitrat, Talkaufschlämmung, Bleomycin, Tetracyclin, Povidon-Iod und einer beliebigen Kombination daraus.

5. Doppelzustandskatheter-Vorrichtung nach Anspruch 1, wobei die Borsten (312, 858) im ersten Zustand durch eine ablösbare Beschichtungssubstanz, ausgewählt aus Hydrogel, einer löslichen Gellösung, Stärke, absorbierbarem Nahtmaterial, wasserlöslichem Klebstoff, denaturierbarem Protein und einer beliebigen Kombination daraus an der äußeren Oberfläche gehalten werden.

## Revendications

1. Dispositif de cathéter double état (300, 800) comprenant :
un corps tubulaire externe allongé (850) comprenant
un tube interne disposé longitudinalement à travers le corps tubulaire externe ;
une partie distale (302, 802) comprenant
une extrémité distale configurée pour insertion dans une cavité corporelle,
une surface externe (310, 856), et
au moins un fenêtrage (806) sur la surface externe ;
une extrémité proximale configurée pour un raccordement à une valve (320, 820);
une structure conductrice (330) configurée pour porter de l'énergie à travers au moins une partie du corps tubulaire externe ; et
des poils (312, 858) ;
dans lequel le dispositif de cathéter est configuré pour effectuer un changement d'un premier état à un second état par introduction d'une énergie sélectionnée dans la structure conductrice, et
dans lequel le premier état comprend le repos sensiblement à plat des poils à la surface externe et dont le second état est **caractérisé par** un changement mécanique comprenant l'extension des poils vers l'extérieur depuis un profil circonférentiel de la surface externe.

2. Dispositif de cathéter double état selon la revendication 1, dans lequel la structure conductrice (330) est choisie parmi les fils noyés, les filaments à semi-conducteur, les lignes à fibre optique, un ruban conducteur co-extrudé, un matériau imprégné d'argent, un canal liquide, un matériau conducteur surmoulé et toute combinaison de ceux-ci.

3. Dispositif de cathéter double état selon la revendication 1, dans lequel les poils (312, 858) comprennent un matériau configuré pour induire une pleurodèse.

4. Dispositif de cathéter double état selon la revendication 3, dans lequel le matériau configuré pour induire une pleurodèse est choisi dans le groupe consistant en le nitrate d'argent, la boue de talc, la bléomycine, la tétracycline, la povidone iodée et toute combinaison de ceux-ci.

5. Dispositif de cathéter double état selon la revendication 1, dans lequel les poils (312, 858) dans le premier état sont tenus à la surface externe par une substance de revêtement libérable choisie parmi un hydrogel, une solution de gel soluble, de l'amidon, un matériau de suture absorbable, un adhésif soluble dans l'eau, une protéine dénaturable et toute combinaison de ceux-ci.
